# EUROPEAN PATENT APPLICATION

(11) **EP 1 402 889 A1**
(43) Date of publication of application: **31.03.2004**
(21) Application number: 02738697.8
(22) Date of filing: 13.06.2002
(51) Int. Cl.: A61K 31/175, A61K 45/00, A61P 43/00

(54) **REMEDIES FOR DISEASES CAUSED BY NONSENSE MUTATION**

(30) Priority: 13.06.2001 US 297533 P
(71) Applicant: Arakawa, Masayuki, Fuchu-shi, Tokyo 183-0051 (JP); Matsuda, Ryouichi, Tokyo 155-0032 (JP)
(72) Inventor: Arakawa, Masayuki, Fuchu-shi, Tokyo 183-0051 (JP); Matsuda, Ryouichi, Tokyo 155-0032 (JP)
(74) Representative: Prins, Adrianus Willem
(86) International application number: PCT/JP2002/005914
(87) International publication number: WO 2002/102361

(57) **Abstract**

The present invention comprises compositions for treating diseases caused by nonsense mutations, including dipeptide antibiotics of Formula (I) shown below, such as negamycin. Unlike aminoglycoside antibiotics such as gentamicin, dipeptide antibiotics can induce the expression of mature proteins by readthrough nonsense mutations without generating serious side effects.

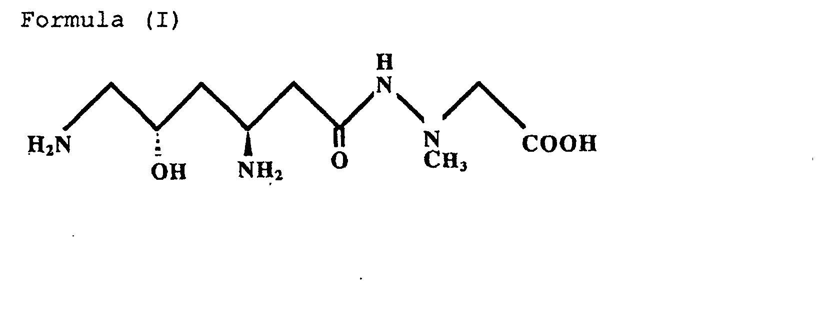

## Description

### Technical Field

The present invention relates to therapeutic agents for treating diseases caused by nonsense mutations, in particular, agents that induce the readthrough of nonsense mutations.

### Background Art

Many genetic diseases are caused by premature stop mutations in human genes, which result in premature termination of translation and the generation of truncated, inactive, and unstable products (Atkinson, J., and Martin, R. (1994) Mutations to nonsense codons in human genetic disease: implications for gene therapy by nonsense suppressor tRNAs. Nucleic Acid. Res. 22, 1327-1334). An example is Duchenne muscular dystrophy (DMD), an X-linked recessive disorder characterized by a lack of dystrophin protein in sarcolemma (plasma membranes of striated muscle fibres), which affects one in 3,500 males.

The mdx mouse is an animal model for DMD used for identifying diseases caused by stop mutations, and for developing methods for treating such diseases. The mdx mouse has a nonsense mutation (CAA to TAA) at the 3,185^{th} nucleotide of the dystrophin gene. This nonsense mutation produces a stop codon at exon 23 (Bulfiled, G., Siller, W. G., Weight, PA., Moore, K. J. (1989) X chromosome-linked muscular dystrophy (mdx) in the mouse. Proc. Natl. Acad. Sci. USA 81, 1189-1192; Sicinski, P., Geng, Y., Ryder-Cook, A. S., Barnard, E. A., Darlison, M. G., Barnard, P. J. (1989) The molecular basis of muscular dystrophy in the mdx mouse. Science 244, 1578-1582) . The stop codon causes premature termination of protein synthesis and thus inhibits the expression of dystrophin and dystrophin-associated glycoprotein complex. This results in a deficiency of these proteins in the muscle cell membrane.

Explicit translocations (mainly, deletions or duplications) of the dystrophin gene are found in 65% of young male patients affected by DMD. However, the remaining 35% have nonsense mutations or other point mutations which affect mRNA splicing. So far, pharmacological therapy for DMD patients and mdx mice has consisted of corticosteroids such as prednisone and deflazacort, or azathioprine, an agent used to reduce corticosteroid use. However, the use of corticosteroids is associated with side effects, and thus they can be used to advantage only for a short time (Granchelli, J. A., Pollina, C., Hudecki, M. S. (2000) Pre-clinical screening of drugs using the mdx mouse. Neuromuscular Disorders. 10, 235-239; Griggs, R. C., Moxley, R. T 3^{rd}., Mendell, J. R., Fenichel, G. M., Brooke, M. H., Pestronk, A., Miller, J. P., Cwik, V. A., Pandya, S., and Robinson, J. (1993) Duchenne dystrophy: randomized, controlled trial of prednisone (18 months) and azathioprine. Neurology 43, 520-527). Thus, identifying a clinically useful method for suppressing premature stop mutations in the dystrophin gene will benefit a significant number of DMD patients.

In recent years, the possibility of chemotherapy which targets nonsense mutations has been gaining strength. Gentamicin (GM) is an aminoglycoside antibiotic that decreases the fidelity of translation and provides a readily accessible treatment for diseases caused by nonsense mutations. GM induces the suppression of stop codons during translation in both prokaryotic and eukaryotic cells. GM is already being used in clinical trials using cells from patients with cystic fibrosis, Hurler's disease, and infant neuronal ceroid lipofuscinosis, all caused by nonsense mutations. Moreover, it is reported that GM restores dystrophin function in drug-treated mdx mice (Barton-Davis, E.R., Cordiner, L., Shoturma, D.I., Leiland, S.E., Sweeney, H.L. (1999) Aminoglycoside antibiotics restore dystrophin function to skeletal muscles of mdx mice. J. Clin. Invest. 104, 375-381). Thus, GM is currently undergoing clinical trials for Duchenne and limb girdle muscular dystrophy.

However, like other aminoglycoside antibiotics, GM tends to cause many side effects such as kidney disorders and hearing loss. Furthermore, the long-term use of a single agent promotes the emergence of bacteria resistant to that agent.

### Disclosure of the Invention

As described above, an aminoglycoside antibiotic such as GM is a drug candidate for treating diseases caused by a nonsense mutation. However, the side effects of GM led the inventors to search for other possible drug candidates distinct from GM, but with a similar stop codon-readthrough activity. Scientific literature reports that negamycin (δ-hydroxy-β-lysine linked with methylhydrazinoacetic acid: NM), a dipeptide antibiotic discovered in Japan in 1970, also induces readthrough of the stop codon in a prokaryotic translation system (Hamada, M., Takeuchi, T., Kondo, S., Ikeda, Y., Naganawa, H., Maeda, K., Okami, Y., and Umezawa, H. (1970) A new antibiotic, negamycin. J: Antibiot. Tokyo 23, 170-171; Uehara, Y., Hori, M., Umezawa, H. (1974) Negamycin inhibits termination of protein synthesis directed by phage f2 RNA in vitro. Biochem. Biophys. Acta. 374, 82-95). The present inventors therefore used mdx mice to analyze whether or not NM can be used as a therapeutic agent for diseases caused by a nonsense mutation. They found that NM restored dystrophin in skeletal muscles both *in vivo* and *in vitro.* NM was found to have an equal or higher readthrough-inducing activity compared to that of GM. In addition to this effect, it also exhibited considerably less toxicity than GM. The present invention is based on these findings made by the present inventors. Specifically, the present invention relates to:
(1) a composition comprising a dipeptide antibiotic for treating a disease caused by a nonsense mutation;
(2) the composition of (1), wherein the dipeptide antibiotic is the compound of Formula (I) shown below, or an analog of said compound that can promote readthrough of the nonsense mutation: 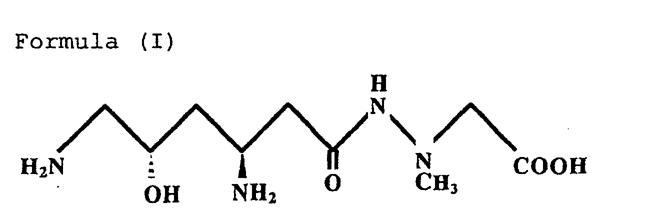 ; and
(3) the composition of (1) or (2) , wherein the disease caused by the nonsense mutation is selected from the group consisting of muscular dystrophy, cystic fibrosis, Hurler's disease, and infantile neuronal ceroid lipofuscinosis.

Such compositions of the present invention for treating diseases caused by nonsense mutations include dipeptide antibiotics. Unlike conventionally used aminoglycoside antibiotics such as gentamicin, dipeptide antibiotics generate no serious side effects, and can promote the readthrough of nonsense mutations. Thus, dipeptide antibiotics can be used instead of or in combination with gentamicin or such, to treat diseases caused by nonsense mutations.

A preferable example of a "dipeptide antibiotic" is negamycin (methyl hydrazinoacetic acid-linked δ-hydroxy-β-lysine: NM) represented by the above formula (I), which has a higher readthrough-promoting activity than gentamicin. The above-mentioned "dipeptide antibiotics" also include compounds structurally similar to negamycin (hereinafter referred to as "negamycin analogs"). As long as a negamycin analog has a structure similar to that of negamycin and has a readthrough-promoting activity similar to that of negamycin, its antimicrobial activity is not relevant.

Negamycin can be prepared, for example, from the culture supernatant of the *Actinomyces* strain M890-C2 or MF752-NF9 (Hamada, M. , Takeuchi, T., Kondo, S. , Ikeda, Y. , Naganawa, H., Maeda, K. , Okami, Y., and Umezawa, H. (1970) Anew antibiotic, negamycin. J. Antibiot. Tokyo 23, 170-171). Negamycin analogs can be prepared from the culture supernatants of the above-mentioned *Actinomyces* strains or other *Actinomyces* strains using, as an index, the activity of promoting the readthrough of nonsense mutations. The negamycin analogs may be naturally-occurring compounds or may be prepared by artificially modifying the above-mentioned negamycin. Such artificial modifications include those introduced for the purposes of: regulating the activity to promote the readthrough of nonsense mutations; formulating drugs; or delivering drugs to target cells (drug delivery); etc.

Further, there is no limitation on the type of "disease caused by a nonsense mutation", as long as it is a disease caused by a genetic deficiency due to a nonsense mutation. Such diseases include, for example, cystic fibrosis (CFTR), thalassemia (β-globin), gastric cancer (APC), hemophilia (Factor VIII, IX), lung cancer, ovarian cancer (p53) or such, Duchenne muscular dystrophy (dystrophin) and limb girdle muscular dystrophy (y-sarcoglycan), obesity (insulin receptor), phenylketonuria (phenylalanine hydroxylase), and such (Atkinson, J., and Martin, R. (1994) Mutations to nonsense codons in human genetic disease: implications for gene therapy by nonsense suppressor tRNAs. Nucleic Acid Res 22: 1327-34). Among the diseases listed above, cystic fibrosis (Howard *et al*. Biochem. Soc. Trans, 21: 846-851 (1996) ; Wilschanski *et al*. Am. J. Respir. Crit. Care Med. 161: 860-865 (2000); Clancy, J. P. *et al*. Am. J. Respir. Crit. Care Med., 163: 1683 (2001)), muscular dystrophy (Barton-Davis *et al*. J. Clin. Invest., 104: 375-381 (1999) ; Wagner, K. R. *et al*., Ann. Neurol. 49: 706-711 (2001)) , Hurler's syndrome (Kim M. Keeling, *et al*. Human Mol. Gene. 10: 291-299 (2001)), and late infantile neural ceroid lipofucinosis (2001 lysosomal tripeptidyl-peptidase 1: Sleat, D. E. *et al*. Europ. J. Paediatr. Neurol. 5 Suppl. A: 57-62 (2001)) have been studied and treated by using the readthrough activity of gentamicin. Therefore, these diseases may be treated with negamycin instead of gentamicin.

The readthrough of nonsense mutations can be induced by administering the above-mentioned negamycin at a daily dose of 1x 10⁻⁷ to 1x 10⁻² mol/kg weight, preferably 1x 10⁻⁶ to 1x 10⁻³ mol/kg weight, over a period appropriate to ensure an effect. Without limitation, powder, granules, tablets, capsules, solutions, injections, and such are used as the dosage form for administration to patients. Thus, a therapeutic composition that comprises a dipeptide antibiotic as an active ingredient, such as the above-mentioned negamycin, can be formulated with adjuvants such as pharmaceutically acceptable excipients, binders, disintegrants, lubricants, flavoring agents, solubilizers, suspending agents, coating agents, and such, as required.

### Brief Description of the Drawings

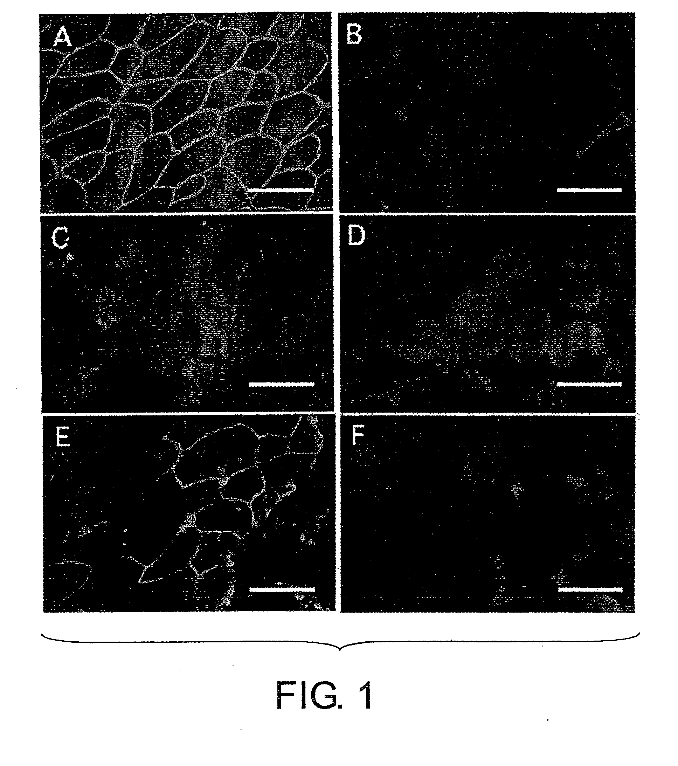

Fig. 1 depicts photographs showing the presence of dystrophin expression and rates of muscle degeneration in Negamycin-treated and untreated mdx TA muscles. Immunofluorescence and EBD stainings were carried out as described in Materials and Methods. Panels A, C, and E are the results of immunofluorescent staining using B10 control mice, untreated mdx mice, and NM-treated mdx mice respectively. Panels B, D, and F are EBD staining patterns of B10 control mice, untreated mdx mice, and NM-treated mdx mice respectively. The bar= 100 µm.

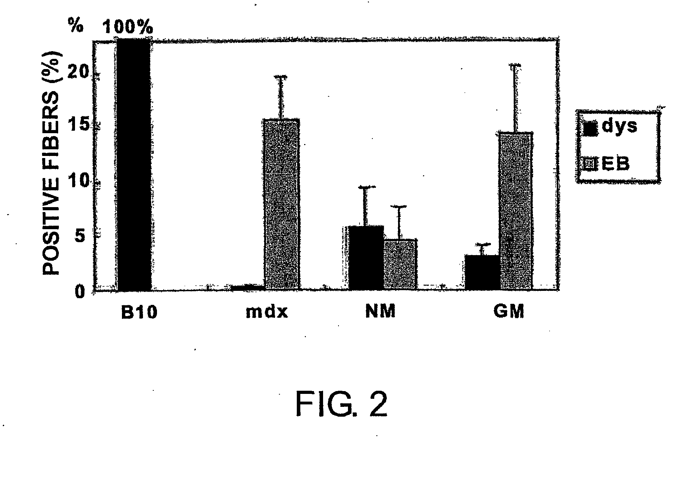

Fig. 2 is a graph that shows the ratio of the expression level of dystrophin (immunofluorescence-positive fiber) and degenerated muscle fiber (EBD dye-positive fiber) in TA muscle fibers of antibiotic-treated mice. The black bar (referred to as "dys" in this figure) indicates the ratio of dystrophin positive fibers; the gray bar (referred to as "EB" in this figure) indicates the ratio of Evans Blue-positive fibers. "NM" indicates mdx mice (seven week-old, six individuals) that were injected with NM in PBS at a dose of 1.2x 10⁻⁵ mol/kg/day subcutaneously for two weeks; "GM" indicates mdx mice (seven week-old, six individuals) that were injected with GM in PBS at a dose of 1.2x 10⁻⁵ mol/kg/day subcutaneously for two weeks. PBS (0.1 ml) alone was injected to control "mdx" mice (n= 6) and C57BL/10 ("B10") (n= 6) mice. About 350-550 muscle fibers were counted in each mouse (n= 6). The bar indicates the mean ± standard deviation.

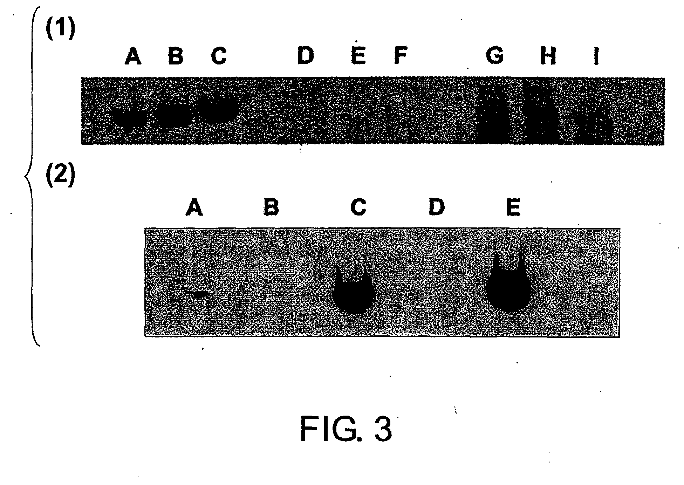

Fig. 3 depicts photographs showing the result of immunoblotting analysis for dystrophin expression. Panel (1) shows immunoblotting results for dystrophin expressed in B10 control mice (lanes A, B, and C) ; control mdx mice (lane D, E, and F) ; and NM-treated mdx mice (lane G, H, and I). Panel (1) shows, from the left, Dystrophin expression in hind leg muscles (lanes A, D, and G), the diaphragm (lanes B, E, and H) , and cardiac muscles (lanes C, F, and I) of each mouse. Panel (2) shows the immunoblotting results for dystrophin expressed in hind leg muscles. Lane A shows NM-treated mdx mice; lane B, a sample buffer; lane C, NM-treated mdx mice (x100 of lane A); lane D, NM-untreated control mdx mice; and lane E, B10 control mice.

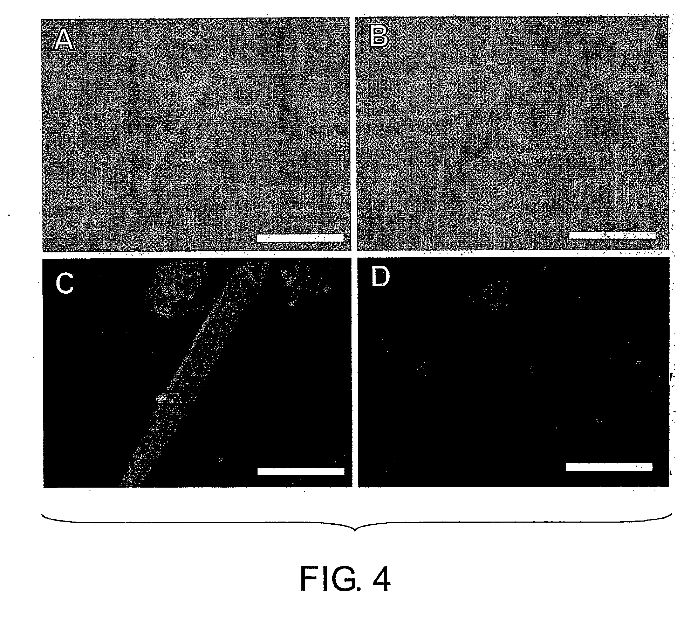

Fig. 4 depicts photographs showing the expression of dystrophin in cultured mdx skeletal muscle cells (mdx-sk). Panels C and D show the expression of dystrophin in the cells presented in panels A and B, respectively. Panel A shows NM (50 µg/ml negamycin)-treated myotubes observed under a phase contrast microscope; panel B, NM-untreated myotubes observed under a phase contrast microscope; panel C, NM (50 µg/ml negamycin)-treated myotubes stained with dystrophin; panel D, NM-untreated myotubes stained with dystrophin. The bar= 40 µm.

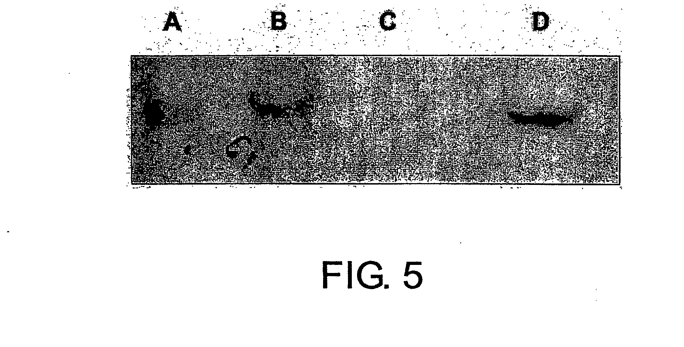

Fig. 5 depicts photographs showing the results of immunoblotting for dystrophin (427 kDa) in cultured mdx skeletal muscle cells (mdx-sk). Lanes A and B show results for myotubes treated with NM (100 µg/ml) for seven days; lane C shows results for untreated mdx myotubes; and lane D for C2C12 myotubes.

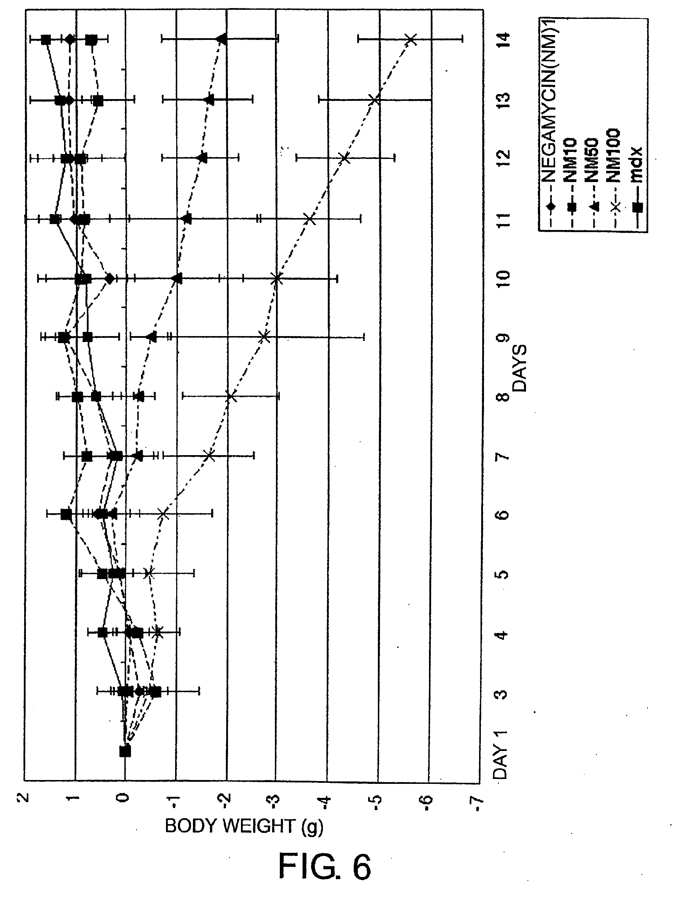

Fig. 6 is a graph that shows weight changes of mdx mice during NM administration. Negamycin-treated mdx mice at a dose of 1.2x 10⁻⁵ mol/kg (NM1: solid diamond); 1.2x 10⁻⁴ mol/kg (NM10: solid square); 6.0x 10⁻⁴ mol/kg (NM50: solid triangle) ; 1.2x 10⁻³ mol/kg (NM100: X) ; and NM-untreated control mdx mice (solid line and solid square).

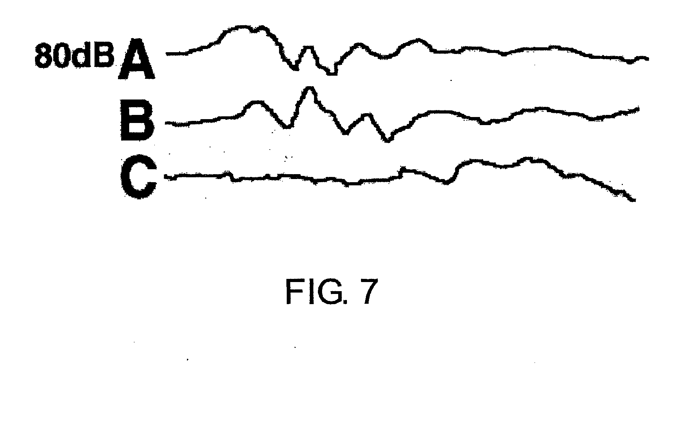

Fig. 7 shows the result of a hearing test based on auditory brain stem response in antibiotic-treated mice. A, B, and C show the results for antibiotic-untreated mice, NM-treated mice and GM-treated mice respectively.

### Best Mode for Carrying out the Invention

The present invention is specifically illustrated below with reference to Examples, but it is not to be construed as being limited thereto.

### [Example 1] Preparation of negamycin

### Culture in flasks:

Cells of the *Actinomyces* M890-C2 strain were inoculated into fifty 500-ml flasks containing 60 ml of C medium (2.0% glucose, 2.0% starch, 2.0% soybean extract, 0.5% dry yeast, 35% CaCO₃, 0.0005% CuSO₄·5H₂O, 0.0005% MnCl₂·4H₂O, 0.005% ZnSO₄·7H₂O) and incubated while shaking (at 220 rpm) at 28°C for four days. The culture media were filtered through (4%) pearlite. The filtrate was collected and subjected to anion-exchange column chromatography (Diaion SA10A, 1.5 liters, OH form). Elution was then carried out with a 0.2 N HCl solution. Fractions retaining antimicrobial activity against the *Escherichia coli* K-12 strain were collected (500-ml fractions; fraction numbers 12-16). The active fractions were neutralized with ammonia water, and then concentrated to 500 ml under reduced pressure. The resulting concentrated solution was then loaded onto a column of anion-exchange resin (Amberlite CG50, 250 ml, NH₄ form) , and eluted with 0.1% ammonia water. Fractions exhibiting antimicrobial activity against the K-12 strain were collected (18-g fractions, fraction numbers 31-70). The active fractions were freeze-dried to give 32.8 mg of a light brown powder. The powder was resuspended in the buffer, and the resulting solution was further loaded onto a column of anion-exchange resins (Amberlite CG50, 250 ml, NH₄ form). Elution was then carried out with 0.1% ammonia water by the same procedure as describe above. Fractions exhibiting antimicrobial activity against the K-12 strain were collected (200-g fractions, fraction number 6). The active fraction was freeze-dried to give 13.8 mg of a white powder.

### Culture in jars:

Cells of the *Actinomyces* M890-C2 strain were inoculated into a jar fermenter containing five liters of C medium. The cells were then cultured while shaking (at 300 rpm) under aerobic conditions of five liters/min at 28°C for five days. Similar to the above-described flask culture, the culture medium was filtered through (4%) pearlite. The filtrate was subjected to anion-exchange column chromatography (Diaion SA10A, 1.5 liters, OH form) , and then active fractions were collected (500-ml fractions; fraction numbers 9-12). The active fractions were neutralized with ammonia water, and concentrated to 500 ml under reduced pressure. The resulting concentrated solution was loaded onto a column of anion-exchange resins. The column was washed with one liter of distilled water. Elution was then carried out with 0.1% ammonia water, and active fractions were collected (200-g fractions, a water-eluted fraction, and ammonia water-eluted fractions 1 and 2). The active fractions were freeze-dried to give 4.8 g of a brown powder. This powder was resuspended in a buffer. The resulting solution was further loaded onto a column of anion-exchange resins (Amberlite CG50, 250 ml, NH₄ form) , and then eluted with 0.1% ammonia water. The active fractions were collected (200-g fractions, fraction numbers 15-16), and freeze-dried to give 27.7 mg of a brown powder. In the second-round purification with anion-exchange resins, fractions 6-9 were found to retain antimicrobial activity.

### [Example 2] Restoration of the expression of dystrophin by the administration of negamycin to a muscular dystrophy mouse model

Mdx mice were used as a dystrophy mouse model. NM was dissolved in PBS and Millipore-filtered just before inj ection, in order to avoid degradation during storage in solution. Mdx mice (male, seven week-old, six for each dose) were injected subcutaneously with an NM solution (137 mM NaCl, 2.68 mM KCl, 8.10 mM Na₂HSO₄, and 1.47 mM KH₂PO₄) prepared using PBS, at half the concentration (1.2x 10⁻⁵ mol/kg) of the solution in the GM experiment by Barton-Davis *et al*. (Barton-Davis, E. R., Cordiner, L. Shoturma, D. I., Leiland, S. E., Sweeney, H. L. (1999) Aminoglycoside antibiotics restore dystrophin function to skeletal muscles of mdx mice. J. Clin. Invest. 104, 375-381) every day for two weeks. Control mdx mice (n= 6) and C57BL/10 (B10, n= 6) mice were injected with PBS (0.1 ml) alone, every day for two weeks.

After injection, immunofluorescence and Evans Blue staining were performed to detect dystrophin. Immunofluoresce staining was carried out as follows using antibodies against the C-terminus of dystrophin. Animals were sacrificed using an overdose of ether gas. The tibialis anterior (TA) muscles were removed and frozen in melting isopentane for immunohistochemistry. Then, 7 µm transverse cryosections were prepared. After pre-incubation in a blocking solution for 15 minutes with 20% horse serum in PBS, the cryosections were washed with PBS three times for ten minutes and incubated for one hour at room temperature with the primary antibody (rabbit anti-dystrophin polyclonal antibody, a gift from Dr. Y. Nonomura, University of Tokyo, 1:200 diluted in PBS containing 2% bovine serum albumin [BSA]). After washing with PBS three times for ten minutes, the sections that reacted with the primary antibody were labeled for one hour with 1:100 diluted fluorescein-labeled anti-rabbit IgG (Amersham Pharmacia Biotech, Tokyo, Japan). After labeling with secondary antibodies, dystrophin was detected by fluorescence microscopy, based on fluorescence emission.

Evans Blue staining was carried out by intraperitoneal injection of Evans Blue Dye (EBD: 2% EBD in PBS, 0.1 ml) to all animals twelve hours before sacrificing. EBD staining visualizes degenerating muscle fibers that have permeable membranes (Matsuda, R., Nishikawa, A., Tanaka, H. *et al*. (1995) Visualization of dystrophic muscle fibers in mdx mice by vital staining with Evans Blue: Evidence of apoptosis in dystrophin-deficient muscle. J. Biochem. 118, 959-964).

As a result of immunofluorescence staining, dystrophin-positive fibers were detected in the NM-treated mdx mice (Fig. 1E) as well as in the dystrophin-positive control B10 mice (Fig. 1A). In contrast, muscle fibers in the NM-untreated mdx mice (Fig. 1C) were all negative for dystrophin (Fig. 1C).

The percentage of dystrophin-positive TA muscle fibers in drug-treated mice was higher than in untreated mdx mice. Among drug-treated mice, the percentage of dystrophin-positive TA muscle fibers in NM-treated mice was higher than in GM-treated mdx mice reported previously (Arakawa, M., Nakayama, Y., Hara, T., Shiozuka, M. , Takeda, S. , Kaga, K., Kondo, S., Morita, S. , Kitamura, T., Matsuda, R. (2001) Negamycin can restore dystrophin in mdx skeletal muscle. Acta. Myologica. XX, 154-158). The percentage of fibers that exhibited increased membrane permeability (EBD-positive fibers) was lower in drug-treated animals than in untreated animals, and further, among drug-treated animals, the percentage was lower in NM-treated mice than in GM-treated mice (Fig. 2).

These results indicated that administration of NM could lead to the restoration of dystrophin expression and effective suppression of the formation of membrane-permeable, degenerated muscle fibers.

As described above, the experiment results obtained by immunofluorescence staining and such showed that NM could restore dystrophin expression. Thus, the expression of dystrophin was confirmed in more detail by immunoprecipitation and immunoblotting.

The left hind leg muscle (600 mg) , the diaphragm (100 mg) , and the cardiac muscle (100 mg) were collected from NM-treated mdx mice, NM-untreated mdx mice, and B10 mice as described above. Each of the specimens was homogenized in 15 ml of a homogenizing solution (pH 7.2) (pyrophosphate mixture, 2 0 mM Na₄P₂O₇, 2 0 mM NaH₂PO₄, and 1mM MgCl₂, pH 7.1) containing 10% sucrose and 0.5 mM EDTA. The homogenization was carried out with a Teflon homogenizer at maximal speed for one minute (Mitchell, R. D., Palade, P., and Fleicher, S. (1983) Purification of morphologically intact triad structures from skeletal muscle. J. Cell Biol. 96, 1008-1016; Yoshida, M., Suzuki, A., Shimizu, T. , and Ozawa, E. (1992) Proteinase-sensitive sites on isolated rabbit dystrophin. J. Biochem. 112, 433-439). The homogenate was centrifuged in an RA rotor (KUBOTA) at 9,000 rpm for 15 minutes. The resulting supernatant was recovered, and then centrifuged again in an RA rotor at 14,000 rpm for 30 minutes to prepare a microsome fraction. The pellet was dissolved in 1 ml of 1% digitonin solution (0.5 M NaCl, 0.5 M sucrose, 0.1 mM PMSF, 50 mM Tris-HCl, and 1U/ml aprotinin, pH7.2).

Immunoprecipitation was performed as described by Abe *et al*. (Abe, M., Saitoh, O., Nakata, H., Yoda, A., and Matsuda, R. (1996) Expression.of neurofilament proteins in proliferating C2C12 mouse skeletal muscle cells. Exp. Cell Res. 229, 48-59) using muscle protein sample preparations prepared from mdx mice and B10 mice as described above. The protein sample preparations described above were each incubated with anti-dystrophin monoclonal antibody DYS2 (10 µl) [Novocastra, Newcastle, UK] at 4°C overnight. Then, wheat germ agglutinin-Sepharose CL-6B (30 µl) (Sigma, Tokyo, Japan) was further added, and the solution was incubated at 4°C for another 60 minutes. After incubation, the solution was centrifuged at 14,000 rpm at 4°C for five minutes. Resulting pellets were washed with 0.2% NP-40 in PBS (500 µl) three times and boiled in sodium dodecyl sulfate (SDS) sample buffer (62.5 mM Tris-HCl, 2% SDS, 5 mM EDTA, 5% 2-mercaptoethanol, 0.1 mM PMSF, and 10% glycerol, pH 6.8) for four minutes. After boiling, the supernatant was collected, and the protein concentration thereof was determined by microburette method.

The samples corresponding to 25 µg of total protein that resulted from the immunoprecipitation were subjected to SDS-polyacrylamide gel electrophoresis (SDS-PAGE) with a 4%-8% gradient gel. After SDS-PAGE, proteins were transferred from the gel onto a nitrocellulose membrane (Gelman Sciences) for immunoblotting. The membrane was immersed in a blocking solution (5% skim milk in 25 mM Tris-HCl [pH 7.4], 137 mM NaCl, 2.68 mM KCl, [TBS], 0.05% Tween20: 5% skim milk TBST or PBST) at 4°C overnight. After blocking, the membrane was incubated with a dystrophin-specific antibody (1:100 diluted anti-dystrophin monoclonal antibody DYS3 or DYS2, [Novocastra, Newcastle, UK], or 1:500 diluted rabbit anti-dystrophin polyclonal antibody; dilutions done using the blocking solution) at room temperature for one hour. After being washed with 5% skim milk TBST or PBST three times for ten minutes, the membrane was incubated with a horseradish peroxidase-conjugated secondary antibody that had been diluted 1:1000 or 1:3000 in 5% skim milk TBST and then washed with TBST or PBST three times for 30 minutes. The washed membrane was treated with an enzyme chemiluminescence (ECL) kit (Amersham Pharmacia Biotech, Tokyo, Japan), and then exposed to an X-ray film, Hyper-film ECL (Amersham Pharmacia Biotech, Tokyo, Japan), for visualizing the electrophoresis pattern of dystrophin.

As a result of immunoblotting, dystrophin bands were detected in hind leg muscles (Fig. 3 (1) A, B, and C) of positive control B10 mice as expected. Also, dystrophin bands were detected in hind leg muscles of NM-treated mdx mice (Fig. 3 (1) G, H, and I) although the band intensity thereof was lower than that of the positive control. From the results of dystrophin expression analysis in hind legs, the dystrophin expression level restored by NM administration (Fig. 3 (2) A) was estimated to be about 10% of the dystrophin expression level in normal B10 hind leg muscle (Fig. 3 (2) E).

### [Example 3] Dystrophin restoring effect of NM in immortalized cells (mdx-sk) derived from mdx skeletal muscle cell

The SV40T immortalized mdx satellite cell line, mdx-sk, was newly established from the mdx mouse to test the dystrophin restoring level in cultured mdx skeletal muscle cells. The mdx-sk line was established by introducing a retroviral vector carrying a cDNA for the temperature-sensitive form of the Simian Virus 40 large T antigen (a kind gift from Dr. Drinkwater) to a primary culture of mdx myoblasts obtained from the mdx mice. The retrovirus was produced using the packaging cell line, Plat-E, as previously described (Morita, S., Kojima, T., Kitamura, T. (2000) Plat-E: an efficient and stable system for transient packaging of retroviruses. Gene Therapy 7, 1063-1066). The cell line was cultured and maintained in Dulbecco's Modified Eagle Medium (DMEM)-high glucose (4,500 mg/l) supplemented with 20% fetal calf serum at 32.5°C in a CO₂ incubator. To induce muscle differentiation, the culture medium was changed to a differentiation medium (10% horse serum in Eagle MEM) and the culture temperature was shifted to 39.5°C.

After induction of differentiation, the cells were cultured, and NM (50 µg/ml (2.0x 10⁻⁴ mol/kg) or 100 µg/ml (4.0x 10⁻⁴ mol/kg) in a differentiation medium solution) was added to the culture. Then, the cells were allowed to differentiate by culturing in the medium without an antibiotic for seven days. C2C12 cells were maintained in a growth medium, and then cultured in a differentiation medium at 38°C in a CO₂ incubator.

In order to examine whether NM can restore dystrophin expression in the mdx-sk cells established as described above, NM was added at a concentration of 50 µg/ml or 100 µg/ml to a differentiation medium of myotubes cultured for seven days, and then culture was continued for another seven days. After culturing (in the presence of 50 µg/ml NM), the restoration of dystrophin expression was investigated by immunofluorescence staining. After being collected from the medium, the mdx-sk cells were washed twice with PBS. The cells were then fixed with 100% ethanol for 15 minutes, and treated with a PBS solution containing 0.5% Triton X-100 for ten minutes. The cells were then washed three times with PBS for ten minutes, and pre-incubated in a blocking solution with PBS containing 20% horse serum for 15 minutes. After being washed three times with PBS for ten minutes, the cells were incubated with a primary antibody (rabbit anti-dystrophin polyclonal antibody (a kind gift from Dr. Nonomura, Tokyo University) diluted 1:200 in PBS containing 2% bovine serum albumin (BSA)) at room temperature for one hour. After being washed three times with PBS for ten minutes, the cells were incubated for labeling with 1:100 diluted fluorescein-labeled anti-rabbit IgG (Amersham Pharmacia Biotech, Tokyo, Japan) at room temperature for one hour. The cells were then observed under a fluorescence microscope.

According to the results of immunofluorescence staining, there was no significant fluorescence signal, and therefore, dystrophin was not expressed in NM-untreated myotubes (Fig. 4B and 4D). On the other hand, strong fluorescence signals were detected in NM-treated myotubes, and therefore, dystrophin was confirmed to be expressed therein (Fig. 4A and 4C).

Furthermore, dystrophin expression in the above-mentioned cultured cells (in the presence of 100 µg/ml (4.0x 10⁻⁴ mol/kg) NM) was also examined by immunoblotting. First, proteins were prepared by homogenizing Mdx-sk myotubes and C2C12 myotubes pooled on ice in 10-cm gelatin-coated plates containing 500 µl of a homogenization solution (HS: 20 mM Tris-HCl (pH 7.6), 150 mM NaCl, 1% Nonidet P-40 (NP-40) , 100 µg/ml DNase, 1mM phenylmethyl sulfonyl fluoride (PMSF) , 1 µg/ml N-tosyl-L-phenylalanyl chloromethyl ketone (TPCK), 1 µg/ml N-tosyl-L-lysyl chloromethyl ketone (TLCK), 200 U/ml aprotinin, and 5 mM ethylenediamine tetra-acetic acid (EDTA)).

Immunoprecipitation was performed according to the method described by Abe *et al*. (supra) as Example 2, using mds-sk myotube protein preparations as above. After centrifugation of mdx-sk and C2C12 myotube protein preparations at 9,000 rpm, the supernatant was collected and incubated with the anti-dystrophin monoclonal antibody DYS2 (3 µl) or the anti-dystrophin monoclonal antibody MANDRA1 (3 µl) (Sigma, Tokyo, Japan) at room temperature (RT) for 60 minutes. Protein A-Sepharose CL-4B (30 µl) (Sigma, Tokyo, Japan) was added, and the solution was incubated at room temperature for another 60 minutes and centrifuged at 14,000 rpm for five minutes. Pellets were washed with 0.2% NP-40 in PBS (500 µl) three times and boiled in sodium dodecyl sulfate (SDS) buffer for five minutes.

A sample corresponding to the total protein amount after immunoprecipitation from each of the 10-cm plates was subjected to SDS-polyacrylamide gel electrophoresis (PAGE) on a 4%-8% gradient gel. After PAGE, the proteins were transferred onto a nitrocellulose membrane (Gelman Sciences) by the same method as described in Example 2. Then, the membrane was subjected to immunoblotting with specific antibodies (1:100 diluted anti-dystrophin monoclonal antibodies DYS3 and DYS2 (Novocastra, Newcastle, UK); 1:500 diluted rabbit anti-dystrophin polyclonal antibody. The electrophoretic pattern of dystrophin was finally visualized by exposing the membrane to X-ray film.

According to the pattern of dystrophin expression visualized by immunoblotting, no band was detected at the position corresponding to 427 kDa in the NM-untreated mdx-sk myotube sample, but a clear band was detected at the position of 427 kDa in the NM-treated mdx-sk myotube sample (Fig. 5A and 5B), as well as in the dystrophin-positive C2C12 myotube sample (Fig. 5D).

### [Example 4] NM toxicity test

Generally, aminoglycoside antibiotics like GM are accompanied by strong side effects. Although these antibiotics are routinely used for the treatment of bacterial infections, they often cause nephrotoxicity and ototoxicity. The present inventors examined NM toxicity by measuring changes in body weight and hearing activity in drug-treated mice.

In order to measure changes in body weight due to drug administration, male mdx mice (seven weeks old, four for each dose) were inj ected daily with NM at 1.2x 10⁻⁵ mol/kg (1x the effective dose), 1.2x 10⁻⁴ mol/kg (10x), 6.0x 10⁻⁴ mol/kg (50x), or 1.2x 10⁻³ mol/kg (100x) for 14 days. The body weight of each mouse was measured everyday. Other mdx mice (seven weeks old, two for each dose) were injected with GM (1.2x 10⁻³ mol/kg [100x]) as a control test. Body weight was also measured every day.

For the ototoxicity assay, each Mdx mouse to be tested was injected with 1.2x 10⁻⁴ mol/kg NM or GM daily for seven days. One day after the final injection, the hearing threshold was measured based on auditory brain stem response, according to the method of Shapiro *et al*. (Shapiro, S. M., Moller, A. R., Shiu, G. K. Brain-stem auditory evoked potentials in rats with high-dose pentbarbital. (1984) Electroencephalogr. Clin. Neurophysiol. 58, 266-276).

According to evaluation of the auditory brain stem response, a hearing loss of 80 dB or lower was detected only in GM-treated mice, and not in NM-treated mice (Fig. 7). The measurement of change in body weight revealed that body weight of mice treated with lower doses of NM (1x and 10x minimal effective dose) increased sequentially (Fig. 6, NM1 and NM10), in a manner similar to control mice. However, the body weight of mice treated with higher doses of NM (50x and 100x) decreased (Fig. 6, NM50 and NM100). It is noteworthy that the highest dose of NM treatment was not lethally toxic. In contrast, the high dose of GM used conventionally (1.2x 10⁻³ mol/kg/day [100x]), killed all of the tested mice within four hours (data not shown). These results suggest that NM has a significantly lower toxicity than GM.

### Industrial Applicability

As described above, the present invention revealed that it is possible to restore dystrophin expression deficiencies that occur due to nonsense mutations. The level to which dystrophin expression was restored by the composition of the present invention was revealed to be higher than with the previously used gentamicin. In addition, unlike gentamicin, the composition of the present invention generated no serious side effects. Thus, the composition of the present invention can be used effectively, either in place of or in combination with gentamicin, as a therapeutic agent for diseases caused by nonsense mutations, such as muscular dystrophy, cystic fibrosis, and Hurler's syndrome.

## Claims

1. A composition comprising a dipeptide antibiotic for treating a disease caused by a nonsense mutation.

2. The composition of claim 1, wherein the dipeptide antibiotic is the compound of Formula (I) shown below, or an analog of said compound that can promote readthrough of the nonsense mutation.

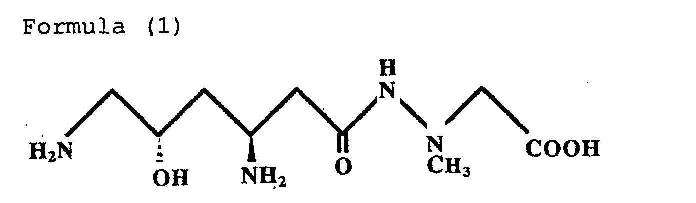

3. The composition of claim 1 or 2, wherein the disease caused by the nonsense mutation is selected from the group consisting of muscular dystrophy, cystic fibrosis, Hurler's disease, and infantile neuronal ceroid lipofuscinosis.
